# EUROPEAN PATENT APPLICATION

(11) **EP 4 644 361 A1**
(43) Date of publication of application: **05.11.2025**
(21) Application number: 23912684.0
(22) Date of filing: 13.12.2023
(51) Int. Cl.: C07C 229/16, C07D 295/15, C07D 295/13, A61K 9/51, A61K 48/00, A61K 31/7088, A61K 39/00

(54) **LIPID NANOPARTICLE FORMULATION COMPRISING IONIZED LIPIDS WITH BRANCHED STRUCTURE, AND USE THEREOF**

(30) Priority: 29.12.2022 KR 20220189895; 13.11.2023 KR 20230156562
(71) Applicant: SurgiNex Co., Ltd., Seoul 06591 (KR)
(72) Inventor: LEE, Hyukjin, Seoul 06557 (KR); JEONG, Michaela, Seoul 02531 (KR); KIM, Minjeong, Seoul 05119 (KR); PARK, Jeongeun, Seoul 05307 (KR); LEE, Yeji, Seongnam-si, Gyeonggi-do 13643 (KR); IM, Seongeun, Daegu 42760 (KR); JUNG, Hyein, Seoul 05090 (KR); KIM, Dokeun, Cheongju-si, Chungcheongbuk-do 28160 (KR); KIM, Si Hyun, Cheongju-si, Chungcheongbuk-do 28160 (KR); KIM, You-Jin, Cheongju-si, Chungcheongbuk-do 28160 (KR); YEO, Jinah, Cheongju-si, Chungcheongbuk-do 28160 (KR); JEON, Ji Hayng, Cheongju-si, Chungcheongbuk-do 28160 (KR); HWANG, Yun-Ho, Cheongju-si, Chungcheongbuk-do 28160 (KR)
(74) Representative: Abel & Imray LLP
(86) International application number: PCT/KR2023/020529
(87) International publication number: WO 2024/144009

(57) **Abstract**

The present invention relates to: ionized lipids comprising lipids with a branched structure; a lipid nanoparticle formulation using same; and use thereof. The ionized lipids of the present invention are a biodegradable lipid material with a lipid structure in which a heteroamine structure is branched, and the lipid nanoparticles using the ionized lipids can deliver a nucleic acid drug and the like with high efficiency, and thus can be effectively used in related technical fields such as mRNA vaccines and therapeutic agents.

## Description

### BACKGROUND OF THE INVENTION

### 1. Field of the Invention

### Cross Reference to Related Application

This application claims the benefit of Application No. 10-2022-0189895, filed December 29, 2022, and Application No. 10-2023-0156562, filed November 13, 2023, which are incorporated by reference herein in their entirety.

### Field of the Invention

The present invention relates to a lipid nanoparticle formulation comprising ionized lipids with a branched structure, and a use thereof.

### 2. Description of the Related Art

In the pharmaceutical industry, Drug Delivery System (DDS), which is designed to efficiently deliver the required amount of drugs while reducing the side effects of drugs and maximizing their efficacy and effectiveness, is a high-value core technology with a high potential for success that can create economic benefits comparable to new drug development, and aims to improve the quality of patient care through efficient drug administration.

Nucleic acids such as siRNA and mRNA are substances that can control the expression of specific proteins in the body, and are receiving attention as important tools for the treatment of cancer, genetic diseases, infectious diseases, and autoimmune disorders. Nucleic acids are large molecular weight anionic substances that are difficult to deliver directly into cells and are easily degraded by enzymes in the blood, so a lot of research is being conducted to overcome these problems.

Until now, the method of transporting nucleic acids into cells by mixing them with positively charged lipids or polymers (called lipid-DNA conjugate (lipoplex) and polymer-DNA conjugate (polyplex), respectively) has been mainly used. Lipid-DNA conjugates are widely used at the cellular level because they bind to nucleic acids and deliver nucleic acids into cells well. However, when injected locally in vivo, they often cause inflammation in the body, and when injected intravascularly, they accumulate mainly in tissues such as lungs, liver, and spleen, which are first-pass organs.

The initially developed ionizable lipid 1,2-dilinoleoyl-3-dimethylaminopropane (DLinDAP) had low gene transfer efficiency and required large amounts of siRNA to achieve therapeutic effects. Afterwards, through structural changes in the amine head group and linker, approximately 300 types of ionized lipids were screened in a mouse blood coagulation factor FVII model, and an optimized ionized lipid (Dlin-MC3-DMA) with a very low ED₅₀ value of 0.005 mg/kg was selected [Acc. Chem. Res. 2019]. This is approximately 1000 times more effective than existing ionized lipids and was used as a delivery vehicle for Onpattro^{®}, the siRNA treatment for hereditary amyloidosis (hATTR), which was world's first approved by the FDA in 2018.

The first mRNA lipid nanoparticle vaccine has been developed by Moderna and Pfizer to overcome the COVID-19 pandemic. Currently, vaccinations have been successfully conducted in several countries, including the United States and Israel. Moderna and Pfizer's coronavirus vaccines use mRNA lipid nanoparticles. The ionized lipid in the Moderna vaccine is SM-102 (Arbutus) {{9-Heptadecanyl 8-{(2-hydroxyethyl)[6-oxo-6-(undecyloxy)hexyl]amino}octanoate}}, and the ionized lipid in the Pfizer vaccine is ALC-0315 (Genevant) {[(4-hydroxybutyl)azanediyl]di(hexane-6,1-diyl) bis(2-hexyldecanoate)}.

Ionized lipids are structures with a tertiary amine head group with varying the degree of ionization depending on the pH in the body and a long hydrocarbon tail connected by a linker, and can surround nucleic acid drugs due to their degree of ionization, which varies depending on the pH. These ionized lipids are then formulated with other components to form a solid lipid nanoparticle structure with a nucleic acid drug encapsulated inside. Ionized lipids are cationic and surround nucleic acid drugs during the low pH formulation process, and at physiological pH they have a neutral surface charge and form lipid nanoparticles by hydrophobic interactions. Lipid nanoparticles introduced into endosomes through endocytosis become cationic again as the endosomal pH decreases, interact with anionic lipids in the endosomal membrane, and are able to release the enclosed nucleic acid drug into the cell via endosomal escape.

As prior arts, Korean Patent Publication No. 10-2020-0040586 discloses the preparation of ionized lipids with 6-membered heterocyclic amine and 1,2-epoxydodecane into lipid nanoparticles for in vivo drug delivery, and Korean Patent Publication No. 10-2022-0103968, and No. 10-2022-0103968 disclose various ionizable lipids and lipid nanoparticle compositions.

Accordingly, the present inventors have made great efforts to develop novel particles having excellent drug encapsulation efficiency and capable of efficiently delivering anionic drugs, nucleic acids, and the like to target organs or cells. As a result, the present invention was completed by preparing lipid nanoparticles including ionized lipids with a branched structure, and confirming that the lipid nanoparticles can encapsulate nucleic acid drugs with high efficiency and induce immunity by the encapsulated nucleic acids.

### SUMMARY OF THE INVENTION

It is an object of the present invention to provide a novel ionizable lipid compound having a branched structure.

It is another object of the present invention to provide lipid nanoparticles comprising the ionizable lipid compound.

It is another object of the present invention to provide a composition for drug delivery comprising the lipid nanoparticles and anionic drugs, nucleic acids, or a combination thereof.

It is another object of the present invention to provide a use of the composition for drug delivery system comprising the lipid nanoparticles and anionic drugs, nucleic acids, or a combination thereof.

It is another object of the present invention to provide a method of drug delivery comprising a step of administering the composition for drug delivery comprising the lipid nanoparticles and anionic drugs, nucleic acids, or a combination thereof to a subject.

To achieve the above objects, the present invention provides an ionizable lipid compound represented by the following [Formula 1] or a pharmaceutically acceptable salt thereof: 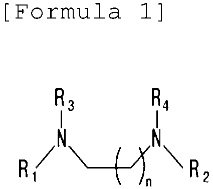
R₁ and R₂ are each independently anyone selected from -H, -C₁₋₁₀ alkyl, -Y, or -C₁₋₁₀ alkyl-NR^{A}R^{B},
R₃ and R₄ are each independently anyone selected from -H, -C₁₋₁₀ alkyl, -Y, or -C₁₋₁₀ alkyl-NR^{A}R^{B}, or connected to each other to form 4- to 8-membered heterocycloalkyl with two N elements,
however, at least one of R₁ to R₄ is -C₁₋₁₀ alkyl-NR^{A}R^{B},
R^{A} and R^{B} are each independently -H, -C₁₋₁₀ alkyl, or -Y,
n is an integer between 0 and 6,
at least one -Y substituent is present,
Y is represented by the following [Formula 2],
when multiple Ys exist, they can be different, 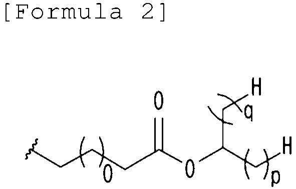
o, p, and q are each independently any integer from 1 to 12.

The present invention also provides a use of an ionizable lipid compound represented by [Formula 1] or a pharmaceutically acceptable salt thereof in the preparation of lipid nanoparticles for drug delivery.

The present invention also provides a use of an ionizable lipid compound represented by [Formula 1] or a pharmaceutically acceptable salt thereof in the preparation of drug delivery system comprising the lipid nanoparticle.

The present invention also provides lipid nanoparticles comprising an ionizable lipid compound or a pharmaceutically acceptable salt thereof.

The present invention also provides a composition for drug delivery comprising the lipid nanoparticles and anionic drugs, nucleic acids, or a combination thereof.

The present invention also provides a use of the composition comprising the lipid nanoparticles and anionic drugs, nucleic acids, or a combination thereof as a drug delivery system.

In addition, the present invention provides a method of drug delivery comprising a step of administering the composition for drug delivery comprising the lipid nanoparticles and anionic drugs, nucleic acids, or a combination thereof to a subject.

### ADVANTAGEOUS EFFECT

The present invention relates to an ionized lipid comprising lipids with a branched structure, a lipid nanoparticle formulation using the same, and a use thereof. The ionized lipid of the present invention is a biodegradable lipid material with a lipid structure in which a heteroamine structure is branched, and the lipid nanoparticles using the ionized lipid can deliver a nucleic acid drug and the like with high efficiency, and thus can be effectively used in related technical fields such as mRNA vaccines and therapeutic agents.

### BRIEF DESCRIPTION OF THE DRAWINGS

Figure 1 is a diagram showing the synthetic process of a novel ionizable lipid with a branched structure.
Figure 2 is a diagram showing the MS spectrum results to confirm the synthesis of ionizable lipids.
Figure 3 is a graph showing the luminescent gene expression levels and cytotoxicity of ionizable lipid nanoparticles in Hela cells.
Figure 4 is a graph showing the luminescent gene expression levels and cytotoxicity of ionizable lipid nanoparticles in HEK 293 cells.
Figure 5 is a graph showing the AST levels in mouse blood after administration of EW244-E-7 lipid nanoparticles.
Figure 6 is a graph showing the ALT levels in mouse blood after administration of EW244-E-7 lipid nanoparticles.
Figure 7 is a diagram showing the bioluminescence of mFluc-loaded EW221-E-7 lipid nanoparticles injected intravenously into mice.
Figure 8 is a diagram showing the bioluminescence of mFluc-loaded EW244-E-7 lipid nanoparticles injected intravenously into mice.
Figure 9 is a diagram showing the bioluminescence of mFluc-loaded EW221-E-7 lipid nanoparticles injected intramuscularly into mice.
Figure 10 is a diagram showing the bioluminescence of mFluc-loaded EW244-E-7 lipid nanoparticles injected intramuscularly into mice.
Figure 11 is a graph showing the expression levels of EPO and MCP-1 after injection of hEPO mRNA-loaded EW244-E-7 lipid nanoparticles into mice.
Figure 12 is a graph showing the expression levels of MCP-1 after injection of hEPO mRNA-loaded EW244-E-7 lipid nanoparticles into mice.
Figure 13 is a graph showing the neutralizing antibody titer after injection of EW244-E-7 lipid nanoparticles loaded with COVID-19 spike mRNA into mice.
Figure 14 is a graph showing the induction of IFN-gamma after injection of EW244-E-7 lipid nanoparticles loaded with COVID-19 spike mRNA into mice.
Figure 15 is a graph showing the neutralizing antibody titer against Wuhan strain (S) after injection of lipid nanoparticles loaded with in-silico spike mRNA into mice.
Figure 16 is a graph showing the neutralizing antibody titer against Indian strain (Delta) after injection of lipid nanoparticles loaded with in-silico spike mRNA into mice.
Figure 17 is a graph showing the neutralizing antibody titer against Omicron (BA.5) after injection of lipid nanoparticles loaded with in-silico spike mRNA into mice.
Figure 18 is a graph showing the induction of IFN-gamma after injection of lipid nanoparticles loaded with in-silico spike mRNA into mice.
Figure 19 is a graph showing the IgG antibody titer after injection of EW244-E-7 lipid nanoparticles loaded with mRNA encoding RSV into mice.

### DESCRIPTION OF THE PREFERRED EMBODIMENTS

Hereinafter, the present invention is described in detail.

The present invention provides an ionizable lipid compound represented by the following [Formula 1] or a pharmaceutically acceptable salt thereof: 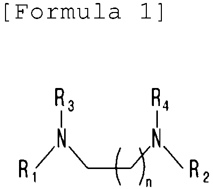
R₁ and R₂ are each independently anyone selected from -H, -C₁₋₁₀ alkyl, -Y, or -C₁₋₁₀ alkyl-NR^{A}R^{B},
R₃ and R₄ are each independently anyone selected from -H, -C₁₋₁₀ alkyl, -Y, or -C₁₋₁₀ alkyl-NR^{A}R^{B}, or connected to each other to form 4- to 8-membered heterocycloalkyl with two N elements,
however, at least one of R₁ to R₄ is -C₁₋₁₀ alkyl-NR^{A}R^{B},
R^{A} and R^{B} are each independently -H, -C₁₋₁₀ alkyl, or -Y,
n is an integer between 0 and 6,
at least one -Y substituent is present,
Y is represented by the following [Formula 2],
when multiple Ys exist, they can be different, 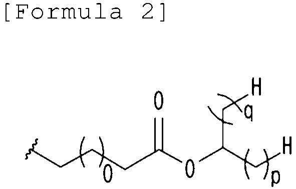
o, p, and q are each independently any integer from 1 to 12.

As another embodiment of the present invention,
in Formula 1 above,
R₁ and R₂ are each independently anyone selected from -H, -C₁₋₆ alkyl, -Y, or -C₁₋₆ alkyl-NR^{A}R^{B},
R₃ and R₄ are each independently anyone selected from -H, -C₁₋₆ alkyl, -Y, or -C₁₋₆ alkyl-NR^{A}R^{B}, or connected to each other to form 4- to 8-membered heterocycloalkyl with two N elements,
however, at least one of R₁ to R₄ is -C₁₋₆ alkyl-NR^{A}R^{B},
R^{A} and R^{B} are each independently -H, -C₁₋₆ alkyl, or - Y,
n is an integer between 0 and 4,
there are 2 to 6 -Y substituents,
Y is represented by the following [Formula 2],
each Y can be different, 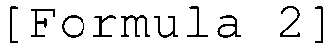 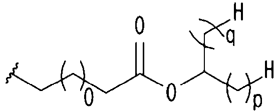
o and p are each independently an integer from 1 to 9, and
q is an integer from 1 to 5.

As another embodiment of the present invention,
In Formula 1 above,
R₁ and R₂ are each independently anyone selected from -H, -C₁₋₄ alkyl, -Y, or -C₁₋₄ alkyl-NR^{A}R^{B},
R₃ and R₄ are each independently anyone selected from -H, -C₁₋₄ alkyl, -Y, or -C₁₋₄ alkyl-NR^{A}R^{B}, or connected to each other to form 5- to 6-membered heterocycloalkyl with two N elements,
however, at least one of R₁ to R₄ is -C₁₋₄ alkyl-NR^{A}R^{B},
R^{A} and R^{B} are each independently -H, -C₁₋₄ alkyl, or - Y,
n is an integer between 1 and 2,
there are 3 to 6 -Y substituents,
Y is represented by the following [Formula 2],
each Y can be different, 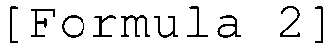 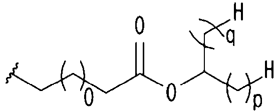
o and p are each independently an integer from 1 to 9, and
q is an integer from 1 to 5.

As another embodiment of the present invention,
In Formula 1 above,
R₁ and R₂ are each independently anyone selected from -H, -C₁₋₄ alkyl, -Y, or -C₁₋₄ alkyl-NR^{A}R^{B},
R₃ and R₄ are each independently anyone selected from -H, -C₁₋₄ alkyl, -Y, or -C₁₋₄ alkyl-NR^{A}R^{B}, or connected to each other to form 5- to 6-membered heterocycloalkyl with two N elements,
however, at least one of R₁ to R₄ is -C₁₋₄ alkyl-NR^{A}R^{B},
R^{A} and R^{B} are each independently -H, -C₁₋₄ alkyl, or - Y,
n is an integer between 1 and 2,
there are 3 to 6 -Y substituents,
Y is represented by the following [Formula 2],
each Y can be different, 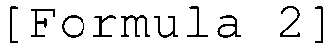 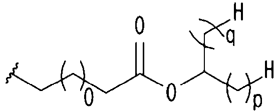
o and p are each independently an integer from 3 to 9, and
q is an integer from 1 to 3.

As another embodiment of the present invention, the compound may be selected from the following compounds:

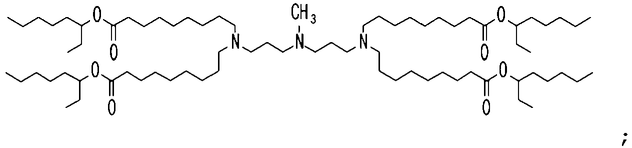

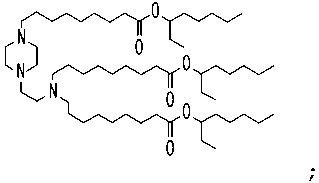

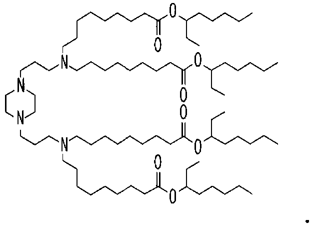

The above ionizable lipid refers to an amine-containing lipid that can be easily protonated, for example, a lipid whose charge state changes depending on the surrounding pH.

The above ionizable lipid can be protonated (positively charged) at pH below the pKa of the cationic lipid, and can be substantially neutral at pH above the pKa.

In the present invention, the ionized lipid is an ionizable compound having lipid-like properties, and serves to encapsulate drugs (e.g., anionic drugs and/or nucleic acids) into lipid nanoparticles with high efficiency through electrostatic interaction with the drugs.

The ionized lipid according to the present invention can be used as the form of a pharmaceutically acceptable salt, in which the salt is preferably acid addition salt formed by pharmaceutically acceptable free acids. Inorganic acids and organic acids can be used as the free acids. Inorganic acids that can be used include hydrochloric acid, bromic acid, sulfuric acid, and phosphoric acid, while organic acids that can be used include citric acid, acetic acid, lactic acid, maleic acid, fumaric acid, gluconic acid, methanesulfonic acid, glycolic acid, succinic acid, tartaric acid, 4-toluenesulfonic acid, galacturonic acid, embonic acid, glutamic acid, and aspartic acid.

The ionized lipid according to the present invention includes not only pharmaceutically acceptable salts, but also all salts, isomers, hydrates and solvates that can be prepared by conventional methods.

The ionizable lipid compound with a branched structure of the present invention can move to the cell membrane and promote endosomal escape by destroying the structure of the cell membrane, or can slow down the rate of hydrolysis or enzymatic degradation of the ester due to the branched structure, so that the drug delivery efficiency is excellent.

The present invention also provides lipid nanoparticles comprising an ionizable lipid compound or a pharmaceutically acceptable salt thereof.

The lipid nanoparticles may further include phospholipid, cholesterol, and lipid-PEG (polyethyleneglycol) conjugates.

The phospholipid plays a role in wrapping and protecting the core formed by the interaction of ionizable lipids and drugs within lipid nanoparticles, and binds to the phospholipid bilayer of target cells to facilitate passage through the cell membrane and endosomal escape during intracellular delivery of the drug.

The phospholipid can be used without limitation as long as the phospholipid can promote the fusion of lipid nanoparticles according to an example. For example, the phospholipid may be at least one selected from the group consisting of dioleoylphosphatidylethanolamine (DOPE), distearoylphosphatidylcholine (DSPC), palmitoyloleoylphosphatidylcholine (POPC), egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), phosphatidylethanolamine (PE), dipalmitoylphosphatidylethanolamine, 1,2-dioleoyl-sn-glycero-3-phosphoethanolamine, 1-palmitoyl-2-oleoyl-sn-glycero-3-phosphoethanolamine (POPE), 1-palmitoyl-2-oleoyl-sn-glycero-3-phosphocholine (POPC), 1,2-dioleoyl-sn-glycero-3-[phospho-L-serine] (DOPS), 1,2-dioleoyl-sn-glycero-3-[phospho-L-serine], etc. Specifically, the phospholipid is DOPE, and the lipid nanoparticles comprising DOPE may be effective for mRNA delivery (excellent drug delivery efficiency for mRNA).

The cholesterol imparts morphological rigidity to the lipid charge within the lipid nanoparticles, and is dispersed in the core and surface of the nanoparticles to enhance the stability of the nanoparticles.

The lipid-PEG (polyethyleneglycol) conjugate, lipid-PEG, PEG-lipid, or lipid-PEG refers to a form in which lipid and PEG are conjugated, and means a lipid to which a polyethylene glycol (PEG) polymer, which is a hydrophilic polymer, is bound to one end. The lipid-PEG conjugate contributes to the stability of nanoparticles in serum within lipid nanoparticles and plays a role in preventing aggregation between nanoparticles. In addition, the lipid-PEG conjugate can enhance the stability of nucleic acids in the body by protecting them from degrading enzymes during in vivo delivery, and can increase the half-life of drugs encapsulated in nanoparticles.

In the lipid-PEG conjugate, PEG may be directly conjugated to the lipid or may be bound to the lipid via a linker moiety. Any linker moiety suitable for binding PEG to lipid may be used, including, for example, ester-free linker moieties and ester-containing linker moieties. The ester-free linker moieties include amido (-C(O)NH-), amino (-NR-), carbonyl (-C(O)-), carbamate (-NHC(O)O-), urea (-NHC(O)NH-), disulfide (-S-S-), ether (-O-), succinyl (-(O)CCH₂CH₂C(O)-), succinamidyl (-NHC(O)CH₂CH₂C(O)NH-), ether, disulfide, as well as combinations thereof (e.g., linkers containing both a carbamate linker moiety and an amido linker moiety), but not always limited thereto. The ester-containing linker moieties include, for example, carbonate (-OC(O)O-), succinoyl, phosphate ester (-O-(O)POH-O-), sulfonate ester, and combinations thereof, but not always limited thereto.

The lipid in the lipid-PEG conjugate can be used without limitation as long as it is a lipid that can bind to polyethylene glycol, and phospholipid and/or cholesterol, which are other components of the lipid nanoparticle, may also be used. Specifically, the lipid in the lipid-PEG conjugate can be ceramide, dimyristoylglycerol (DMG), succinoyl-diacylglycerol (s-DAG), distearoylphosphatidylcholine (DSPC), distearoylphosphatidylethanolamine (DSPE), or cholesterol, and more specifically C16-PEG2000 ceramide.

The PEG in the lipid-PEG conjugate is a hydrophilic polymer that has the ability to inhibit the adsorption of plasma proteins, thereby increasing the circulation time of lipid nanoparticles in the body and preventing aggregation between nanoparticles. In addition, the lipid-PEG conjugate can exhibit stealth function in vivo, preventing degradation of nanoparticles.

The lipid nanoparticles may contain ionizable lipid : phospholipid : cholesterol : lipid-PEG conjugate in a molar ratio of 15 to 35:15 to 35:40 to 60:0.1 to 5, preferably in a molar ratio of 25 to 40 : 10 to 25 : 40 to 60 : 0.5 to 3, more preferably in a molar ratio of 25 to 30:17 to 22:50 to 55:1 to 2.

The present invention also provides a composition for drug delivery comprising the lipid nanoparticles and anionic drugs, nucleic acids, or a combination thereof.

The anionic drug may be at least one selected from the group consisting of peptides, protein drugs, protein-nucleic acid constructs, and anionic biopolymer-drug conjugates.

The nucleic acid may be at least one selected from the group consisting of messenger ribonucleic acid (mRNA), small interfering ribonucleic acid (siRNA), ribosomal ribonucleic acid (rRNA), ribonucleic acid (RNA), deoxyribonucleic acid (DNA), complementary deoxyribonucleic acid (cDNA), aptamer, transfer ribonucleic acid (tRNA), guide ribonucleic acid (gRNA), single guide ribonucleic acid (sgRNA), antisense oligonucleotide, shRNA, miRNA, ribozyme, PNA, and DNAzyme.

The composition for drug delivery may comprise a physiologically active substance such as an anionic drug and/or a therapeutic nucleic acid enclosed inside the lipid nanoparticles, wherein the physiologically active substance such as an anionic drug and/or a therapeutic nucleic acid is stably and highly efficiently enclosed, thereby enabling the delivery composition to exhibit an excellent therapeutic effect. In addition, there is an advantage in that the type of drug encapsulated inside the lipid nanoparticles can be varied depending on the therapeutic purpose.

The lipid nanoparticles may have anionic drugs and/or nucleic acids encapsulated therein (in the lipid nanoparticles). The description of the lipid nanoparticles in which anionic drugs and/or nucleic acids are encapsulated (inside the lipid nanoparticle) is the same as described above for the lipid nanoparticles.

The weight ratio of the ionizable lipid and the drug (anionic drug, nucleic acid, or combination thereof) contained in the lipid nanoparticles may be 1 to 20:1, preferably 1 to 15:1, 1 to 10:1, and more preferably 7.5 to 10:1.

In the present invention, the composition for drug delivery can be used as a pharmaceutical composition for preventing and treating diseases.

The pharmaceutical composition may be administered systemically or locally, and specifically by a delivery route selected from the group consisting of intradermal, subcutaneous, intramuscular, intraocular, intraarticular, intraventricular, intrathecal, oral, intravenous, intratracheal, intraperitoneal, intranasal, intrauterine delivery, or any combination thereof.

The pharmaceutical composition is administered in a pharmaceutically effective dose. In the present invention, the term "pharmaceutically effective dose" means an amount sufficient to treat a disease with a reasonable benefit/risk ratio applicable to medical treatment, and the effective dose level can be determined according to the factors including disease type and severity, drug activity, sensitivity to drug, time of administration, administration route and excretion rate, duration of treatment, concomitant drugs, and other factors well known in the medical field. The pharmaceutical composition according to one example may be administered as an individual therapeutic agent or in combination with other therapeutic agents, may be administered sequentially or simultaneously with conventional therapeutic agents, and may be administered singly or in multiple doses. Taking all of the above factors into consideration, it is important to administer the amount that can achieve the maximum effect with the minimum amount without side effects, and this can be easily determined by those skilled in the art. Specifically, the pharmaceutically effective dose according to the present invention may vary depending on the age, gender, and weight of a patient and may be administered daily, every other day, or divided into 1 to 3 times a day. However, since the dose can be increased or decreased depending on the route of administration, severity of obesity, gender, weight, age, etc., the dose does not limit the scope of the present invention in any way.

The present invention also provides a use of the composition comprising the lipid nanoparticles and anionic drugs, nucleic acids, or a combination thereof as a drug delivery system.

In addition, the present invention provides a method of drug delivery comprising a step of administering the composition for drug delivery comprising the lipid nanoparticles and anionic drugs, nucleic acids, or a combination thereof to a subject.

The subject includes all mammals including humans, non-human primates, dogs, cats, horses, sheep, goats, cows, rabbits, pigs, rats and mice, but not always limited thereto. The administration may be systemic or local administration and specifically may be selected from the group consisting of intradermal, subcutaneous, intramuscular, intraocular, intraarticular, intraventricular, intrathecal, oral delivery, intravenous, intratracheal, intraperitoneal, intranasal, intrauterine delivery, or any combination thereof.

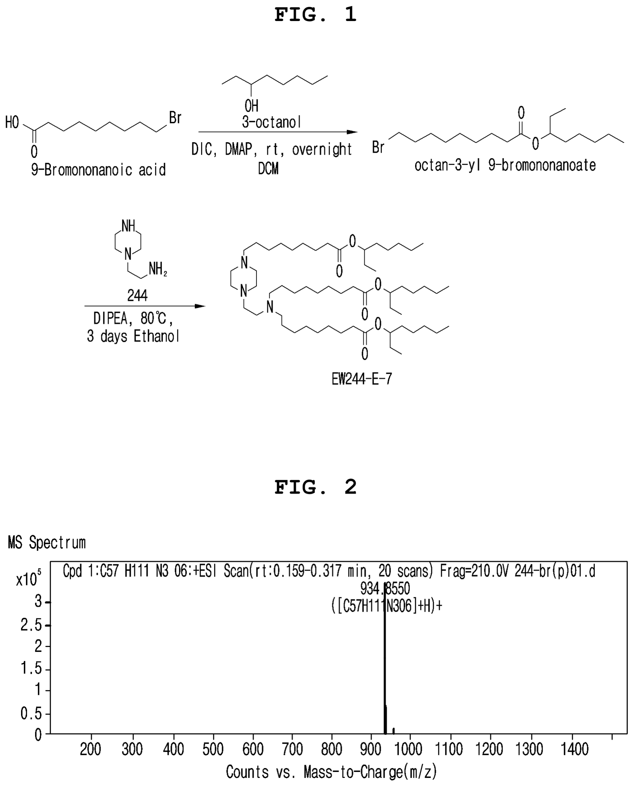

In specific examples and experimental examples, the present inventors synthesized ionizable lipids with biodegradable functional groups introduced into the heteroamine structure (see Figure 1) and confirmed that the ionizable lipids were well synthesized (see Figure 2). In addition, cholesterol, phospholipids, and lipid-PEG conjugates were dissolved in the synthesized ionizable lipids, and lipid nanoparticles encapsulated with nucleic acids were prepared by mixing the lipids and mRNA in a volume ratio of 10:1. The prepared nucleic acid-loaded lipid nanoparticles exhibited good particle size for drug release, uniform, and excellent drug loading rates.

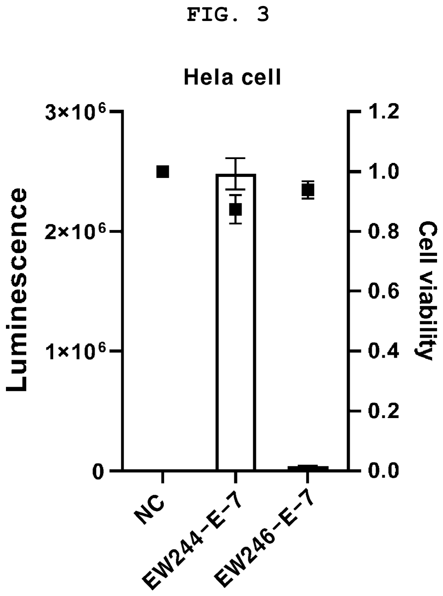

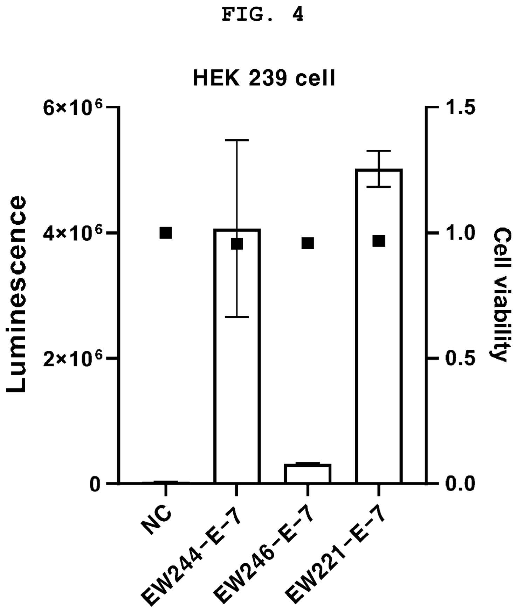

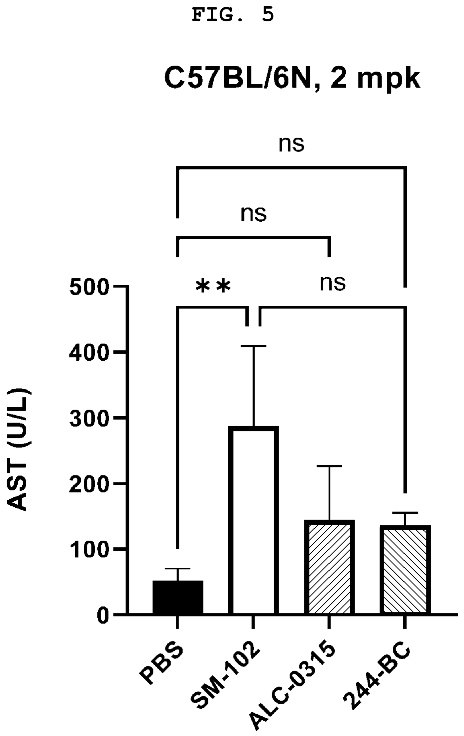

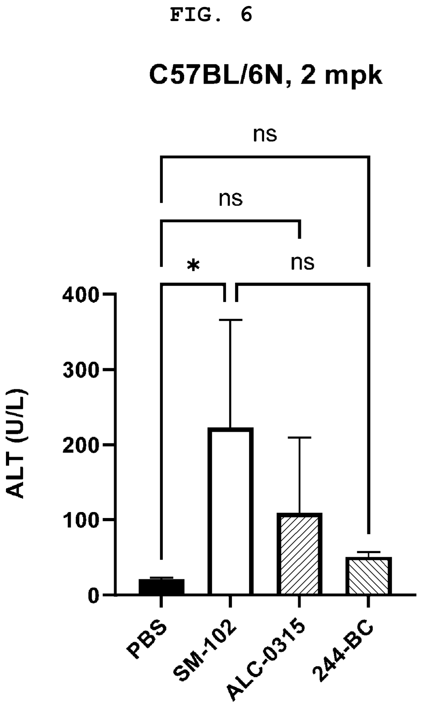

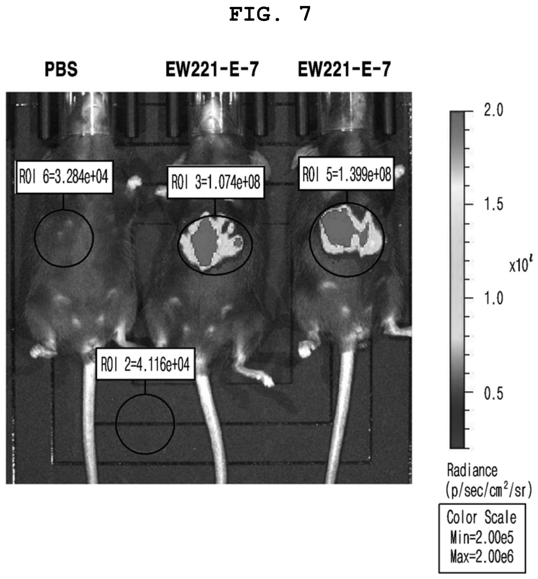

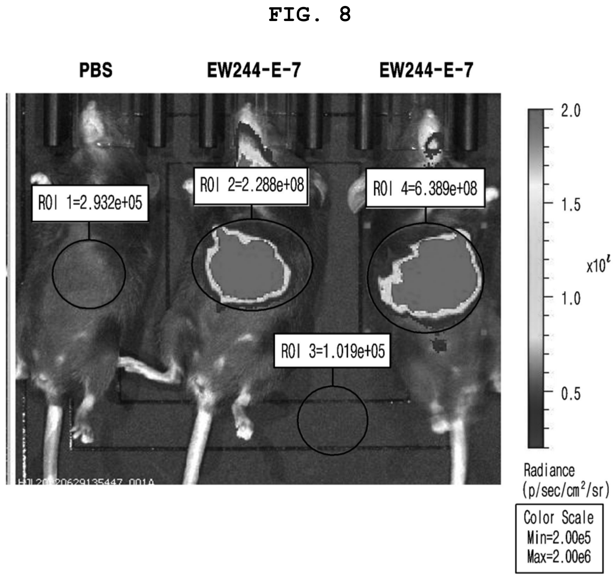

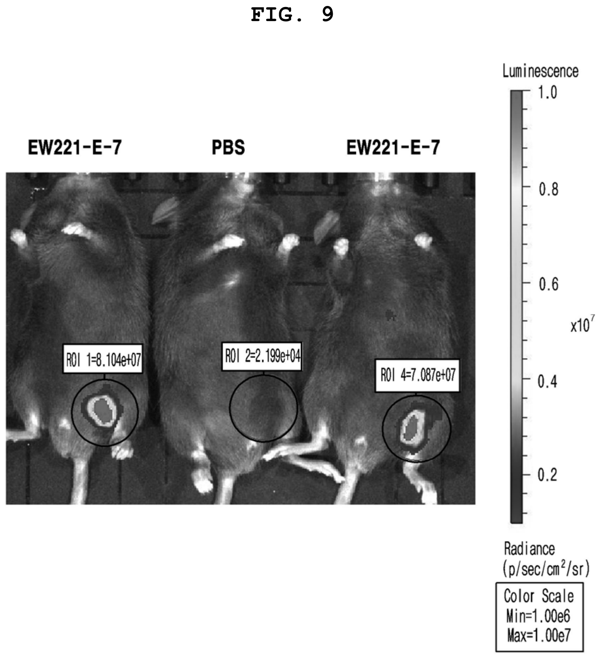

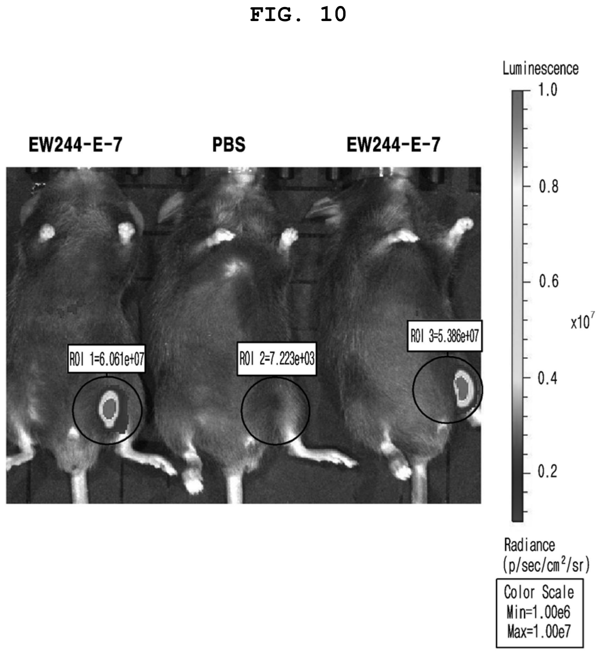

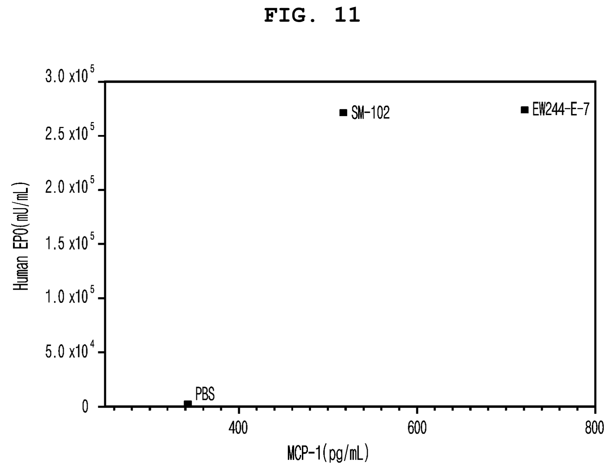

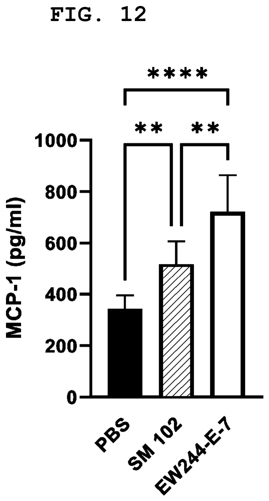

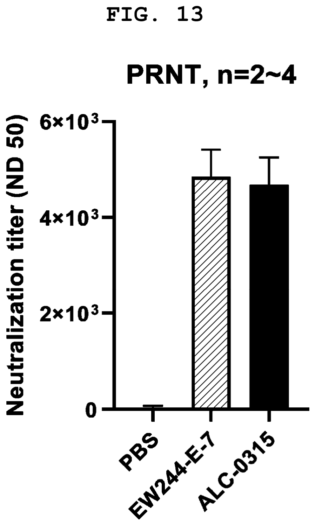

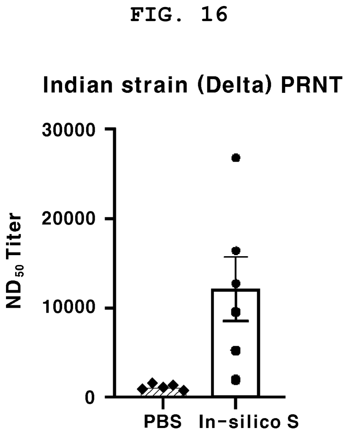

Next, the luminescence of the lipid nanoparticles loaded with mFluc was measured to confirm the excellent intracellular nucleic acid delivery efficiency and absence of cytotoxicity (Figures 3 and 4). In addition, the lipid nanoparticles were administered to mice and confirmed that they did not cause liver toxicity in the animals (Figures 5 and 6). In addition, it was confirmed that the lipid nanoparticles loaded with mFluc were delivered to the liver by intravenous injection into mice (Figures 7 and 8) and that the lipid nanoparticles were delivered to the injection site by intramuscular injection (Figures 9 and 10), indicating that the lipid nanoparticles are suitable for in vivo drug delivery. In addition, the present inventors delivered the lipid nanoparticles loaded with hEPO mRNA to mice, and confirmed that they were suitable for protein expression in vivo and had excellent initial immune-generating ability (Figures 11 and 12). Furthermore, the lipid nanoparticles loaded with COVID-19 mRNA or mRNA encoding respiratory syncytial virus were delivered to mice and found to increase the levels of neutralizing antibody titers, IFN-gamma secretion, and IgG titers (Figures 13 to 19), thereby confirming that the lipid nanoparticles induced humoral or cellular immune responses.

From the above results, it can be seen that the lipid nanoparticles comprising the ionized lipids with a branched structure of the present invention can deliver nucleic acid gene therapeutics and vaccines with high efficiency, and thus can be effectively used in lipid nanoparticle-mediated mRNA vaccines, gene therapy, and other related technical fields.

### Embodiments for Carrying Out the Invention

### Example 1: Synthesis of ionizable lipids

### <1-1> Synthesis of ionizable lipids

Biodegradable functional groups were introduced into the heteroamine structure to synthesize ionizable lipids with a branched structure.

Specifically, 9-bromononanoic acid, DIC (1.5 equivalents), and DMAP (0.2 equivalents) were added to 3-octanol in DCM solvent and reacted overnight at 25°C (Figure 1). Afterwards, the reactant was purified using a CombiFlash column in Hexane/Ethyl Acetate (5:1 v/v). The solvent was evaporated, and the product was dissolved in ethanol. Then, DIPEA (1 equivalent) and the amine with the formula in Table 1 below (0.3 equivalents) were added thereto and reacted at 25°C for 3 days. The reactant was purified using a CombiFlash column in DCM/MeOH (9:1 v/v). Accordingly, noble ionizable lipids containing various amine head groups and ester bonds were obtained. The obtained ionizable lipids were named EW221-E-7, EW244-E-7, and EW246-E-7 according to the type of amine.

**[Table 1]**

| Name | Amine | Ionizable lipid |
|---|---|---|
| 221 | 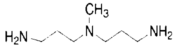 | 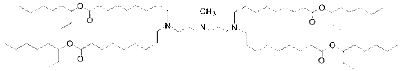 |
| 244 | 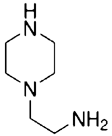 | 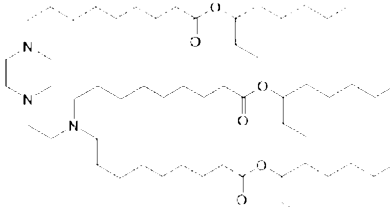 |
| 246 | 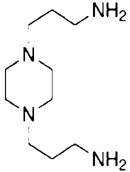 | 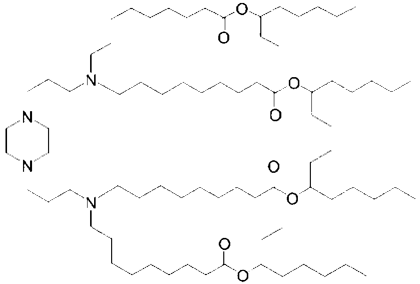 |

### <1-2> Confirmation of synthesized ionizable lipid

To confirm the ionizable lipid synthesized in Example <1-1> above, nuclear magnetic resonance analysis (NMR spectroscopy) was performed.

Specifically, 5 µg of the ionizable lipid (EW244-E-7) synthesized in Example <1-1> was diluted in 0.5 mℓ of CDCl₃ (Sigma, USA) to prepare a concentration of 100 mmole. Then, 0.5 mℓ of the lipid solution was added to a 400 MHz NMR tube, capped, and sealed with parafilm to obtain NMR spectra using Agilent 400 MHZ FT-NMR (Agilent, USA).

As a result, as shown in Figure 2, it was found that the signals representing each functional group of EW244-E-7 were saturated.

In addition, mass spectrometry (MS) was performed to identify the synthesized ionizable lipid (EW244-E-7).

Specifically, the ionizable lipid was diluted in ethanol to a concentration of 0.5 ppm or less and analyzed by 6230 LC/MS (Agilent Technologies, Palo Alto, CA, USA) with a Zorbax SB-C18 separation column (Agilent Technologies, 100 mmX2.1 mm i.d., 3.5 µm).

As a result, as shown in Table 2, it was confirmed that the measured mass-to-charge ratio (m/z) and the calculated mass-to-charge ratio of the ionizable lipid (EW244-E-7) were almost same.

**[Table 2]**

| Formula | Calculated m/z | Measured m/z |
|---|---|---|
| C57H111N3O6 | 933.8476 | 933.8550 |

From the above results, it was confirmed that the ionizable lipid was well synthesized.

### Example 2: Preparation of lipid nanoparticles

### <2-1> Preparation of lipid nanoparticles loaded with nucleic acids

The ionizable lipid (EW244-E-7) synthesized in Example <1-1>, phospholipid (DOPE) (Avanti, USA), cholesterol (cholesterol powder, BioReagent, suitable for cell culture, ≥99%, Sigma, Korea), and lipid-PEG conjugate (C16-PEG2000 ceramide (Avanti, USA) were dissolved in ethanol at a molar ratio of 26.5:20:52:1.5 (Table 3). mRNA was dissolved in 10 mM sodium citrate (Sigma, Korea) buffer. Ethanol containing ionized lipid, cholesterol, phospholipid, and lipid-PEG dissolved therein and citric acid buffer were mixed in a volume ratio of 1:3, respectively, through a microfluidic mixing device (Benchtop Nanoassemblr; PNI, Canada) at a flow rate of 12 mℓ/min to prepare nucleic acid-loaded lipid nanoparticles (LNP).

**[Table 3]**

| Molar ratio (%) | EW244-E-7 lipid nanoparticles |
|---|---|
| Ionized lipid (EW244-E-7) | 26.5 |
| Phospholipid (DOPE) | 20 |
| Cholesterol | 52 |
| Lipid-PEG (ceramide C16 PEG) | 1.5 |

**[Table 4]**

| Weight ratio | EW244-E-7 lipid nanoparticles |
|---|---|
| Lipid/mRNA | 10 |

### <2-2> Physicochemical properties of nucleic acid-loaded lipid nanoparticles

### <2-2-1> Measurement of particle size

The size of the mRNA-loaded lipid nanoparticles synthesized in Example <2-1> above was measured.

Specifically, the firefly luciferase mRNA (mFluc, SEQ. ID. No: 1) contained in the EW244-E-7 lipid nanoparticles synthesized in Example <2-1> above was diluted with PBS to a concentration of 1 µg/mℓ, and the diameter, polydispersity index (PDI), and surface charge (zeta potential) of the lipid nanoparticles were measured using dynamic light scattering (DLS) on Malvern Zetasizer Nano (Malvern Instruments, UK).

As a result, as shown in Table 5, the EW244-E-7 lipid nanoparticles exhibited excellent particle size for drug release, and the particles were found to be uniform.

### <2-2-2> Measurement of drug encapsulation efficacy

Ribogreen assay was performed to measure the nucleic acid drug encapsulation efficacy.

Specifically, for the Ribogreen assay (Quant-iT^{™} RiboGreen^{®} RNA, Invitrogen), the lipid nanoparticles loaded with nucleic acid drugs were diluted with 50 µℓ of 1xTE buffer to a final concentration of RNA of 4 ~ 7 µg/mℓ in a 96-well plate. To the group not treated with Triton-X (Triton-x LNP(-)), 50 µℓ of 1xTE buffer was added, and to the group treated with Triton-X (Triton-X LNP(+)), 50 µℓ of 2% Triton-X buffer was added. The above mixture was incubated at 37°C for 10 minutes to disaggregate the lipid nanoparticles with Triton-X and release the encapsulated nucleic acids. 100 µℓ of Ribogreen reagent was added to each well. The fluorescence intensity (FL) of Triton LNP(-) and Triton LNP(+) was measured with a wavelength bandwidth (excitation: 485 nm, emission: 528 nm) on Infinite^{®} 200 PRO NanoQuant (Tecan). The drug encapsulation efficacy (%) was calculated as follows. Drug encapsulation efficacy(%) = (Fluorescence intensity of Triton LNP(+) - Fluorescence intensity of Triton LNP(-))/(Fluorescence intensity of Triton LNP(+)) X 100

As a result, as shown in Table 5, it was confirmed that the EW244-E-7 lipid nanoparticles can encapsulate drugs with high efficiency.

**[Table 5]**

| **EW244-E-7 lipid nanoparticles** | |
|---|---|
| Diameter (nm) | 92.17 |
| PDI | 0.131±0.06 |
| Surface charge (mV) | -3.14~-3.92 |
| Drug encapsulation efficacy (%) | 91.4 |

### Experimental Example 1: Intracellular nucleic acid delivery and cytotoxicity of lipid nanoparticles

An experiment was conducted to confirm the intracellular nucleic acid delivery efficiency and cytotoxicity using the mFluc-encapsulated lipid nanoparticles prepared in Example <2-1>.

Specifically, luminescence was measured 24 hours after treatment of 20 ng of the mFluc-encapsulated lipid nanoparticles to Hela cells or HEK293 cells.

As a result, as shown in Figure 3, in Hela cells, the EW244-E-7 lipid nanoparticles showed higher expression effects compared to other lipid nanoparticles. In addition, as shown in Figure 4, high expression effects of the EW221-E-7 and EW244-E-7 lipid nanoparticles were confirmed in HEK293 cells. It was also confirmed that the EW221-E-7 and EW244-E-7 lipid nanoparticles had almost no toxicity.

### Experimental Example 2: Confirmation of liver toxicity of lipid nanoparticles

An experiment was conducted to confirm the toxicity in animals by administering the mFluc-encapsulated lipid nanoparticles prepared in Example <2-1> to mice.

AST (aspartate aminotransferase) and ALT (alanine aminotransferase) are values that can measure the presence of diseases such as liver cell disease or hepatitis. They are usually present in the blood at low levels, but when liver cells are damaged, they are released and the concentration in the blood may increase. Therefore, they can be used as indicators for confirming liver toxicity.

Specifically, the mRNA-encapsulated lipid nanoparticles were intravenously administered to 7-week-old C57BL/6 mice at a dose of 2 mg/kg based on mRNA. 24 hours after administration, blood samples were collected to measure the AST and ALT levels in the blood. As the controls, SM-102, an ionized lipid of the Moderna vaccine, and ALC-0315, an ionized lipid of the Pfizer vaccine, were used.

**[Table 6]**

| Injection (2 mg/kg) | AST (mean±SD, U/L) | ALT (mean±SD, U/L) |
|---|---|---|
| PBS | 51.75±18.63 | 21.25±1.89 |
| SM-102 | 287.75±121.81 | 223.25±142.59 |
| ALC-0315 | 144.75±82.25 | 109.50±100.06 |
| 244-BC | 136.00±19.00 | 50.67±6.66 |

As a result, as shown in Table 6 and Figures 5 and 6, mice administered with EW244-E-7 lipid nanoparticles showed lower levels of liver toxicity compared to mice administered with Moderna's SM-102 or Pfizer's ALC-0315. This suggests that the EW244-E-7 lipid nanoparticles are very safe.

### Experimental Example 3: Confirmation of in vivo expression of lipid nanoparticles

### <3-1> Delivery of lipid nanoparticles via intravenous injection

First, mFluc-encapsulated lipid nanoparticles were prepared and their physicochemical properties were confirmed.

As a result, as shown in Table 7, the size of the mFluc-encapsulated EW221-E-7 and EW244-E-7 lipid nanoparticles was confirmed to be efficient for drug delivery, the particles were uniform, and the drug encapsulation efficacy was excellent.

**[Table 7]**

| | EW221-E-7 lipid nanoparticles | EW244-E-7 lipid nanoparticles |
|---|---|---|
| Hit | 7.60 ×10⁷ | 5.72 ×10⁷ |
| Size | 88.29nm | 99.47nm |
| PDI | 0.227 | 0.177 |
| Drug encapsulation efficacy | 60.6% | 87.1% |

Next, the mFluc-encapsulated lipid nanoparticles were delivered to mice via intravenous injection, and bioluminescence was observed to confirm the drug delivery efficiency *in vivo.*

Specifically, 2 ug of the mFluc-encapsulated EW221-E-7 and EW244-E-7 lipid nanoparticles were intravenously injected into 7-week-old C57BL/6 mice, and 0.25 mg/kg of luciferin was administered intraperitoneally 3 hours later, and bioluminescence was confirmed using IVIS (PerkinElmer, USA) equipment.

As a result, as shown in Figures 7 and 8, it was confirmed that most of the EW221-E-7 and EW244-E-7 lipid nanoparticles were delivered to the liver.

The above results suggest that *in vivo* drug delivery is possible by intravenous administration of the lipid nanoparticles.

### <3-2> Delivery of lipid nanoparticles via intramuscular injection

First, mFluc-encapsulated lipid nanoparticles were prepared and their physicochemical properties were confirmed.

As a result, as shown in Table 8, the size of the mFluc-encapsulated EW221-E-7 and EW244-E-7 lipid nanoparticles was confirmed to be efficient for drug delivery, the particles were uniform, and the drug encapsulation efficacy was excellent.

**[Table 8]**

| | EW221-E-7 lipid nanoparticles | EW244-E-7 lipid nanoparticles |
|---|---|---|
| Hit | 7.60 ×10⁷ | 5.72 ×10⁷ |
| Size | 88.29nm | 99.47nm |
| PDI | 0.227 | 0.177 |
| Drug encapsulation efficacy | 60.6% | 87.1% |

Next, the mFluc-encapsulated lipid nanoparticles were delivered to mice via intramuscular injection, and bioluminescence was observed to confirm the drug delivery efficiency *in vivo.*

Specifically, 2 ug of the mFluc-encapsulated EW221-E-7 and EW244-E-7 lipid nanoparticles were intramuscularly injected into 7-week-old C57BL/6 mice, and 0.25 mg/kg of luciferin was administered intraperitoneally 3 hours later, and bioluminescence was confirmed using IVIS (PerkinElmer, USA) equipment.

As a result, as shown in Figures 9 and 10, it was confirmed that most of the EW221-E-7 and EW244-E-7 lipid nanoparticles were delivered to the injection site.

The above results suggest that *in vivo* drug delivery is possible by intramuscular administration of the lipid nanoparticles.

### Experimental Example 4: Confirmation of initial immune-generating ability of lipid nanoparticles

First, lipid nanoparticles encapsulated with hEPO (human EPO) mRNA (SEQ. ID. NO: 2) were prepared, and as a control, the same mRNA was encapsulated in SM-102, an ionized lipid of Moderna vaccine, and their physicochemical properties were compared (Table 9).

**[Table 9]**

| | EW244-E-7 | SM-102 |
|---|---|---|
| Drug encapsulation efficacy | 95.9 | 95.8 |
| Size (nm) | 77.88 | 52.18 |
| PDI | 0.072 | 0.088 |

After delivering the hEPO mRNA-encapsulated nano lipid particles to mice, the concentrations of human EPO and MCP-1 in the blood were measured to confirm the initial protein expression and initial immune-generating ability.

Specifically, 0.5 mg/kg of the hEPO mRNA-encapsulated lipid nanoparticles were intravenously injected into 7-week-old Balb/c mice. After 6 hours, blood was collected to obtain serum, and hEPO and MCP-1 were quantitatively analyzed using hEPO ELISA kit and MCP-1 ELISA kit. SM-102, an ionized lipid in the Moderna vaccine, was used as a control.

As a result, as shown in Figures 11 and 12, when EW244-E-7 and SM-102 were administered, the serum hEPO concentrations were observed to be high at similar levels, and the MCP-1 expression, which is induced by EPO, was observed to be higher when EW244-E-7 was administered than when SM-102 was administered.

The above results suggest that the EW244-E-7 lipid nanoparticles have high protein expression ability and excellent initial immune-generating ability.

### Experimental Example 5: Confirmation of immune-generating ability of lipid nanoparticles

### <5-1> Confirmation of immune-inducing ability against coronavirus

First, lipid nanoparticles encapsulated with COVID-19 spike mRNA (SEQ. ID. NO: 3) were prepared and their physicochemical properties were confirmed. The COVID-19 spike mRNA was provided by Seoul National University, and ALC-0315 was used as a positive control.

As a result, as shown in Table 10, the size of the EW244-E-7 lipid nanoparticles encapsulated with COVID-19 spike mRNA was confirmed to be efficient for drug delivery, the particles were uniform, and the drug encapsulation efficacy was excellent.

**[Table 10]**

| | | ALC-0315 lipid nanoparticles | EW244-E-7 lipid nanoparticles |
|---|---|---|---|
| | Size | 88.29nm | 99.47nm |
| First adminis tration | PDI | 0.227 | 0.177 |
| | Drug encapsulation efficacy | 60.6% | 87.1% |
| Second adminis tration | Size | 47.83nm | 59.65nm |
| | PDI | 0.157 | 0.108 |
| | Drug encapsulation efficacy | 52.1% | 96.3% |

Next, the lipid nanoparticles encapsulated with COVID-19 spike mRNA were delivered to mice, and neutralizing antibody titers and IFN-gamma levels were measured.

Specifically, 0.25 mg/kg of mRNA was firstly administered to 7-week-old Balb/c mice via intramuscular injection. Three weeks later, the second administration was performed in the same manner. Three weeks after the second administration, serum was collected and spleen was extracted, and the neutralizing antibody titers and IFN-gamma levels against SARS-CoV-2 were measured, respectively.

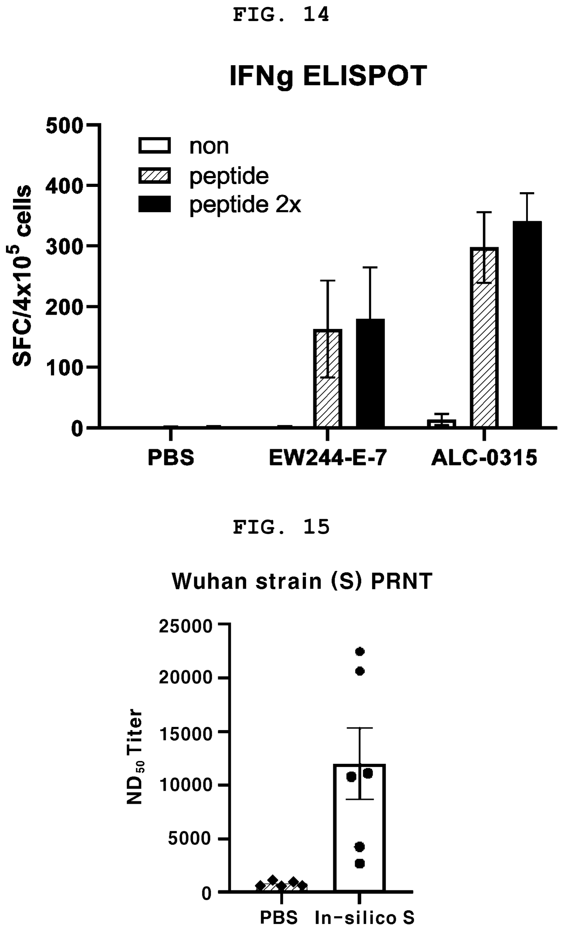

As a result, as shown in Figure 13, it was confirmed that the EW244-E-7 lipid nanoparticles exhibited similar neutralizing antibody titers compared to the positive control, ALC-0315. In addition, as shown in Figure 14, it was confirmed that the IFN-gamma secretion was increased with increasing the peptide concentration in the EW244-E-7 lipid nanoparticles.

The above results suggest that the EW244-E-7 lipid nanoparticles can induce both humoral and cellular immune responses.

### <5-2> Confirmation of immune-inducing ability of COVID 19 universal vaccine

First, lipid nanoparticles encapsulated with in-silico spike mRNA (SEQ. ID. NO: 4) were prepared. After delivering the lipid nanoparticles encapsulated with COVID-19 mRNA to mice, the neutralizing antibody titers and IFN-gamma levels were measured.

Specifically, 0.25 mg/kg of mRNA was firstly administered to 7-week-old Balb/c mice via intramuscular injection. Three weeks later, the second administration was performed in the same manner. Three weeks after the second administration, serum was collected and spleen was extracted, and the neutralizing antibody titers and IFN-gamma levels against SARS-CoV-2 were measured, respectively.

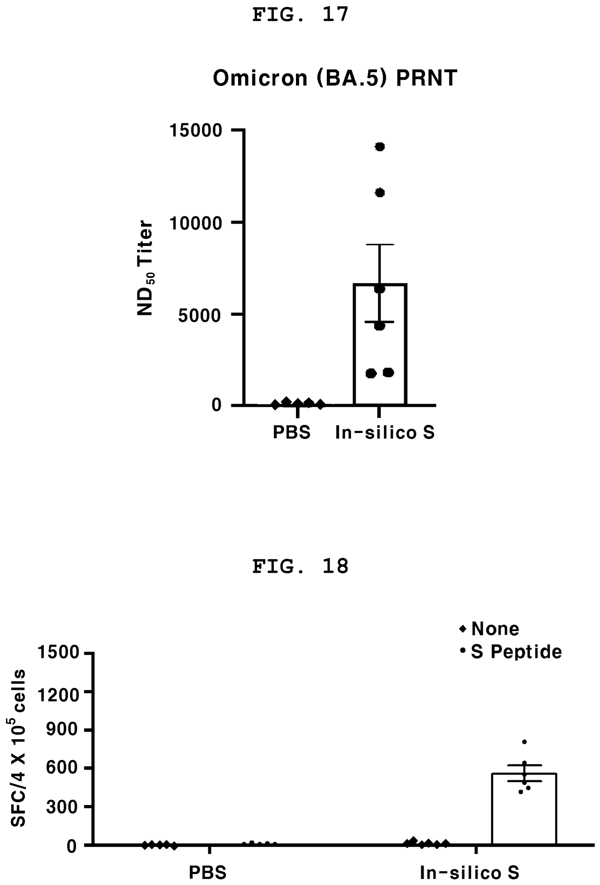

As a result, as shown in Figures 15 to 17, the mice vaccinated with the EW244-E-7 lipid nanoparticles were confirmed to form similar levels of neutralizing antibody titers against Wuhan strain (S), Indian strain (Delta), and Omicron (BA.5), i.e., all COVID-19 strains. In addition, as shown in Figure 18, since the IFN-gamma secretion was confirmed to be high, it was confirmed that the T cell response was active.

The above results suggest that the EW244-E-7 lipid nanoparticles can induce both humoral and cellular immune responses and may have general applicability against various COVID-19 strains.

### <5-3> Confirmation of immune-inducing ability against cellular respiratory syncytial virus

First, lipid nanoparticles encapsulated with mRNA (SEQ. ID. NO: 5) encoding respiratory syncytial virus (RSV) were prepared and their physicochemical properties were confirmed.

As a result, as shown in Table 11, the size of the EW244-E-7 lipid nanoparticles encapsulated with mRNA encoding RSV was confirmed to be efficient for drug delivery, the particles were uniform, and the drug encapsulation efficacy was excellent.

**[Table 11]**

| | | EW244-E-7 lipid nanoparticles |
|---|---|---|
| First administration | Size | 55.49nm |
| | PDI | 0.136 |
| | Drug encapsulation efficacy | 90.0% |
| Second administration | Size | 97.90nm |
| | PDI | 0.010 |
| | Drug encapsulation efficacy | 93.8% |

Next, the lipid nanoparticles encapsulated with mRNA encoding RSV were delivered to mice, and then IgG titers were measured.

Specifically, 0.5 mg/kg of mRNA was firstly administered to 7-week-old Balb/c mice via intramuscular injection. Two weeks later, the second administration was performed in the same manner. One week after the second administration, serum was collected, and the neutralizing antibody titers against RSV were measured. The positive control group [(+) ctrl.] was administered with rAd/3xGmFcm, an adenovirus vector-based vaccine, and the negative control group [(-) ctrl.] was not vaccinated.

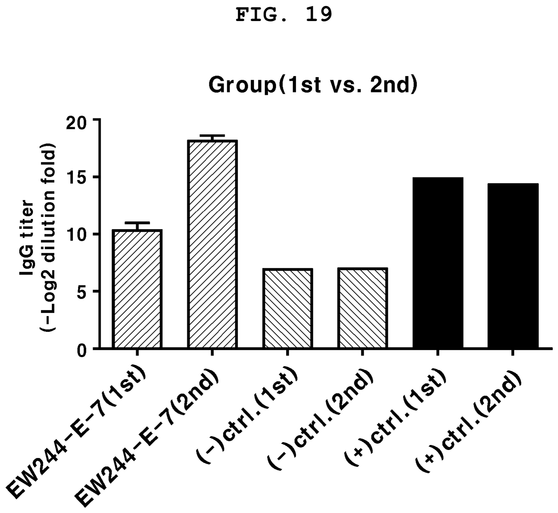

As a result, as shown in Figure 19, after the second administration, IgG titers were higher in the group administered with the EW244-E-7 lipid nanoparticles compared to the positive control group.

The above results suggest that the EW244-E-7 lipid nanoparticles have excellent immune-inducing ability against cellular respiratory syncytial virus.

## Claims

1. An ionizable lipid compound represented by the following [Formula 1] or a pharmaceutically acceptable salt thereof: 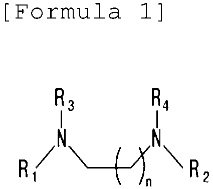
R₁ and R₂ are each independently anyone selected from -H, -C₁₋₁₀ alkyl, -Y, or -C₁₋₁₀ alkyl-NR^{A}R^{B},
R₃ and R₄ are each independently anyone selected from -H, -C₁₋₁₀ alkyl, -Y, or -C₁₋₁₀ alkyl-NR^{A}R^{B}, or connected to each other to form 4- to 8-membered heterocycloalkyl with two N elements,
however, at least one of R₁ to R₄ is -C₁₋₁₀ alkyl-NR^{A}R^{B},
R^{A} and R^{B} are each independently -H, -C₁₋₁₀ alkyl, or -Y,
n is an integer between 0 and 6,
at least one -Y substituent is present,
Y is represented by the following [Formula 2],
when multiple Ys exist, they can be different.

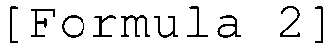

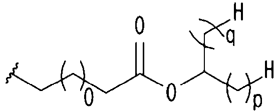

2. The ionizable lipid compound or the pharmaceutically acceptable salt thereof according to claim 1, wherein:
in Formula 1, R₁ and R₂ are each independently anyone selected from -H, -C₁₋₆ alkyl, -Y, or -C₁₋₆ alkyl-NR^{A}R^{B},
R₃ and R₄ are each independently anyone selected from -H, -C₁₋₆ alkyl, -Y, or -C₁₋₆ alkyl-NR^{A}R^{B}, or connected to each other to form 4- to 8-membered heterocycloalkyl with two N elements,
however, at least one of R₁ to R₄ is -C₁₋₆ alkyl-NR^{A}R^{B},
R^{A} and R^{B} are each independently -H, -C₁₋₆ alkyl, or - Y,
n is an integer between 0 and 4,
there are 2 to 6 -Y substituents,
Y is represented by the following [Formula 2],
each Y can be different, 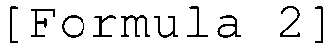 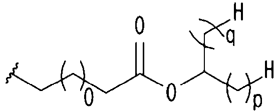
o and p are each independently an integer from 1 to 9, and
q is an integer from 1 to 5.

3. The ionizable lipid compound or the pharmaceutically acceptable salt thereof according to claim 1, wherein:
in Formula 1, R₁ and R₂ are each independently anyone selected from -H, -C₁₋₄ alkyl, -Y, or -C₁₋₄ alkyl-NR^{A}R^{B},
R₃ and R₄ are each independently anyone selected from -H, -C₁₋₄ alkyl, -Y, or -C₁₋₄ alkyl-NR^{A}R^{B}, or connected to each other to form 5- to 6-membered heterocycloalkyl with two N elements,
however, at least one of R₁ to R₄ is -C₁₋₄ alkyl-NR^{A}R^{B},
R^{A} and R^{B} are each independently -H, -C₁₋₄ alkyl, or - Y,
n is an integer between 1 and 2,
there are 3 to 6 -Y substituents,
Y is represented by the following [Formula 2],
each Y can be different, 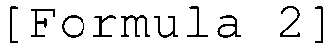 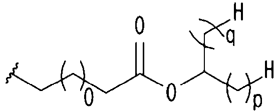
o and p are each independently an integer from 1 to 9, and
q is an integer from 1 to 5.

4. The ionizable lipid compound or the pharmaceutically acceptable salt thereof according to claim 1, wherein:
in Formula 1, R₁ and R₂ are each independently anyone selected from -H, -C₁₋₄ alkyl, -Y, or -C₁₋₄ alkyl-NR^{A}R^{B},
R₃ and R₄ are each independently anyone selected from -H, -C₁₋₄ alkyl, -Y, or -C₁₋₄ alkyl-NR^{A}R^{B}, or connected to each other to form 5- to 6-membered heterocycloalkyl with two N elements,
however, at least one of R₁ to R₄ is -C₁₋₄ alkyl-NR^{A}R^{B},
R^{A} and R^{B} are each independently -H, -C₁₋₄ alkyl, or - Y,
n is an integer between 1 and 2,
there are 3 to 6 -Y substituents,
Y is represented by the following [Formula 2],
each Y can be different, 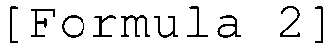 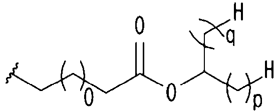
o and p are each independently an integer from 3 to 9, and
q is an integer from 1 to 3.

5. The ionizable lipid compound or the pharmaceutically acceptable salt thereof according to claim 1, wherein the compound is selected from the following compounds:

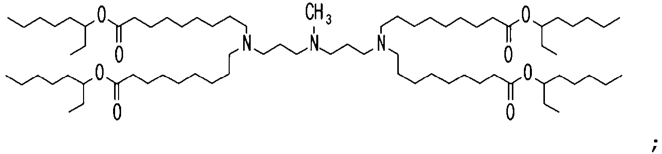

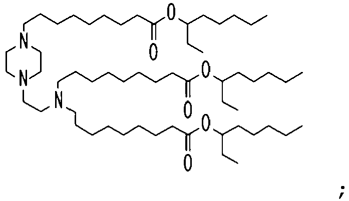

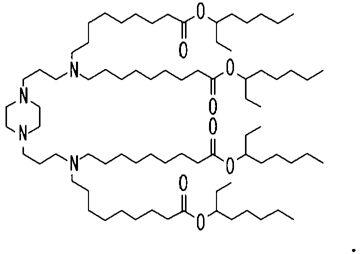

6. A lipid nanoparticle comprising the ionizable lipid compound or the pharmaceutically acceptable salt thereof of any one of claims 1 to 5.

7. The lipid nanoparticle according to claim 6, wherein the lipid nanoparticle further contains phospholipid, cholesterol or lipid-PEG (polyethyleneglycol).

8. The lipid nanoparticle according to claim 7, wherein the lipid nanoparticle comprises ionizable lipid : phospholipid : cholesterol : lipid-PEG conjugate in a molar ratio of 15 to 35 : 15 to 35 : 40 to 60 : 0.1 to 5.

9. The lipid nanoparticle according to claim 8, wherein the phospholipid is at least one selected from the group consisting DOPE, DSPC, POPC, EPC, DOPC, DPPC, DOPG, DPPG, DSPE, Phosphatidylethanolamine, dipalmitoylphosphatidylethanolamine, 1,2-dioleoyl-sn-glycero-3-phosphoethanolamine, POPE, POPC, DOPS, and 1,2-dioleoyl-sn-glycero-3-[phospho-L-serine].

10. The lipid nanoparticle according to claim 8, wherein the lipid in the lipid-PEG conjugate is at least one selected from the group consisting of ceramide, dimyristoylglycerol (DMG), succinoyl-diacylglycerol (s-DAG), distearoylphosphatidylcholine (DSPC), distearoylphosphatidylethanolamine (DSPE), and cholesterol.

11. A composition for drug delivery comprising:
(1) the lipid nanoparticle of claim 6; and
(2) anionic drug, nucleic acid, or a combination thereof.

12. The composition for drug delivery according to claim 11, wherein the anionic drug is at least one selected from the group consisting of peptides, proteins, protein-nucleic acid constructs, and anionic biopolymer-drug conjugates.

13. The composition for drug delivery according to claim 11, wherein the nucleic acid is at least one selected from the group consisting of messenger ribonucleic acid (mRNA), small interfering ribonucleic acid (siRNA), ribosomal ribonucleic acid (rRNA), ribonucleic acid (RNA), deoxyribonucleic acid (DNA), complementary deoxyribonucleic acid (cDNA), aptamer, transfer ribonucleic acid (tRNA), guide ribonucleic acid (gRNA), single guide ribonucleic acid (sgRNA), antisense oligonucleotide, shRNA, miRNA, ribozyme, PNA, and DNAzyme.

14. The composition for drug delivery according to claim 11, wherein the anionic drug, nucleic acid, or a combination thereof is encapsulated inside the lipid nanoparticle.

15. The composition for drug delivery according to claim 11, wherein the composition is administered systemically or locally.
